# EUROPÄISCHE PATENTSCHRIFT

(11) **EP 1 214 102 B1**
(45) Veröffentlichungstag und Bekanntmachung des Hinweises auf die Patenterteilung: **25.06.2003**
(21) Anmeldenummer: 00969273.2
(22) Anmeldetag: 20.09.2000
(51) Int. Cl.: A61L 2/07, A47L 11/34

(54) **VORRICHTUNG ZUR REINIGUNG UND DESINFEKTION VON OBERFLÄCHEN**
DEVICE FOR CLEANING AND DISINFECTING SURFACES
DISPOSITIF DE NETTOYAGE ET DE DESINFECTION DE SURFACES

(30) Priorität: 22.09.1999 DE 19945438
(43) Veröffentlichungstag der Anmeldung: 19.06.2002
(73) Patentinhaber: Beurer, Genoveva, 80997 München (DE); Schäffer, Martin, 80637 München (DE)
(72) Erfinder: KEIM, Bettina, 80335 Munchen (DE); SCHÄFFER, Martin, 80637 München (DE)
(74) Vertreter: Bardehle, Heinz, Dipl.-Ing.
(86) Internationale Anmeldenummer: EP0009216
(87) Internationale Veröffentlichungsnummer: WO01021221

(56) Entgegenhaltungen:
- EP-A- 0 032 654
- GB-A- 2 276 811
- US-A- 5 513 415

## Beschreibung

Die Erfindung betrifft ein Gerät zur Reinigung und anschließenden Desinfektion von Oberflächen, insbesondere Bodenflächen, umfassend eine mechanische Reinigungsvorrichtung und eine wasserdampfaufbringende Desinfektionsvorrichtung.

Um allgemein anerkannten Hygieneanforderungen nachzukommen, werden in Krankenhäusern, Arztpraxen, der Lebensmittelwirtschaft usw. verschiedene maschinelle Techniken zur periodischen Reinigung angewandt und meist durch Zugabe von chemischen Wirkstoffen für eine zusätzliche Desinfektionswirkung ergänzt.

Bekannt ist, dass eine gründliche mechanische Reinigung bereits eine erhebliche Keimreduktion so behandelter Bodenflächen bewirkt.

Eine weitere Reduktion erfolgt durch Auftrag chemischer Desinfektionsmittel, oftmals in Kombination mit Flächenreinigungsmitteln.

Die hohen Konzentrationen der Flächen- und Reinigungsmittel haben zur Folge, dass relativ hohe Rückstände der Wirkstoffe auf den so bearbeiteten Oberflächen zurückbleiben.

Da mehrere Studien einen gesundheitsschädigenden Einfluss auf die Personen nachwiesen, die besonders häufig mit Desinfektionsmitteln in Berührung kommen, z.B. Reinigungs- und Krankenhauspflegepersonal, ist es wünschenswert, den Einsatz solcher Mittel so weit wie möglich zu reduzieren. Hinzu kommen Untersuchungen, wonach die Rückstände von Flächendesinfektionsmitteln durchaus einen Einfluss auf die Entstehung multiresistenter Hospitalismusstämme haben.

Bei Überlegungen zu planvollen und sinnvollen Hygienemaßnahmen wie einer Flächendesinfektion können weder das Wachstum von Mikroorganismen, noch deren Abwehrmechanismen gegen chemische Wirkstoffe isoliert vom Umgebungsmilieu betrachtet werden.

Somit ist ein Verfahren wünschenswert, bei dem der Einsatz chemischer WirkStoffe reduziert werden kann, eine Resistenzentwicklung von Mikroorganismen gegen solche Wirkstoffe ausgeschlossen ist und aus ökologischen und ökonomischen Gründen Reinigungs- und Desinfektions-Komponente in möglichst einem Arbeitsschritt zusammengefasst sind.

Aus der Patentschrift DE 195 04 402 C 1 ist ein fahrbarer Bodenreinigungsautomat bekannt, der außer der Ausstattung mit den üblichen Reinigungsvorrichtungen so verbessert ist, dass auch stark verschmutzte Böden in möglichst kurzer Zeit gereinigt werden können.

Bei diesem fahrbaren Gerät fehlt jedoch eine Desinfektionsvorrichtung, die eine über durch die mechanische Reinigung bewirkte, darüber hinausgehende Desinfektionsleistung, d.h. von deutlich mehr als 80 % Keimreduktion, erreicht.

Aus der Offenlegungsschrift DE 196 44 570 A1 ist ein fahrbares Bodenreinigungsgerät bekannt, bei dem aus Gründen der Gewichtsreduzierung und der Vereinfachung des konstruktiven Aufbaus der Reinigungsflüssigkeitstank als einteiliger, geschlossener Hohlkörper aus Kunststoff ausgestaltet ist, und der Schmutzflüssigkeitsbehälter in Form einer doppelwandigen Aufnahme des Hohlkörpers ausgebildet ist.

Bei diesem fahrbaren Bodenreinigungsgerät könnte eine Desinfektionswirkung allenfalls durch Zugabe von chemischen Desinfektionsmitteln in den genannten Reinigungsflüssigkeitstank erreicht werden, was jedoch aus den obengenannten Gründen der Resistenzbildung von Nachteil ist.

Aus der GB 2 276 811 A ist ein fahrbares Bodenreinigungsgerät bekannt, bei dem zusätzlich zur Reinigung eine Dampferzeugungsvorrichtung vorgesehen ist. Durch den erzeugten Dampf soll der Schmutz vor der Reinigung gelöst werden, womit also das Gerät wegen der nach der Bedampfüng erfolgenden Reinigung nicht zur Desinfektion verwendet werden kann.

Weiter bekannt aus der Offenlegungsschrift DE 195 47 650 A1 ist ein Verfahren zum Desinfizieren von Flächen, wobei einem Wasser-Dampf-Gemisch ein antimikrobieller Wirkstoff zugesetzt wird.

In diesem Verfahren wird vorgeschlagen, einerseits die Konzentration bekannter Flächendesinfektionsmittel, z.B. aus der Gruppe der quartären Ammoniumverbindungen, zu verringern, andererseits die Desinfektionsleistung der Wirkstoffe um ein Vielfaches dadurch zu erhöhen, dass die Wirkstoffe mit Wasser-Dampf gemischt und aufgebracht werden. Bei der Durchführung des erfindungsgemäßen Verfahrens mit handelsüblichen Dampfreinigungsgeräten soll eine befriedigende Desinfektionsleistung durch eine etwa 2-3-malige Behandlung von Bodenflächen erreicht werden.

In der genannten Offenlegungsschrift wird in 3 Vergleichsversuchen festgestellt, dass bei den getesteten handelsüblichen Dampfreinigungsgeräten ohne gleichzeitige Einwirkung von Dampf und antimikrobiellem Wirkstoff selbst bei einer Einwirkzeit von 120 min keine befriedigenden Desinfektionsleistungen erzielt wurden.

In diesem erfindungsgemäßen Verfahren ist jedoch von Nachteil, dass eine Resistenzentwicklung auch bei geringen Wirkstoffkonzentrationen nicht nur nicht ausgeschlossen ist, sondern die Gefahr der Resistenzbildung sogar noch erhöht ist. Weiterhin ist in diesem Verfahren nicht geklärt, was mit dem entstandenen Kondensat aus chemischen Wirkstoffen und Wasser-Dampf-Gemisch entsteht, ob und wie das Kondensat entfernt wird, bzw. wie einer raschen und unvermeidlichen Rekontamination feucht behandelter Oberflächen begegnet werden kann. Problematisch ist weiterhin die Gefahr einer Gesundheitsgefährdung durch Bildung giftiger Dämpfe aus den erwähnten Gemischen, wobei Rückstände von Reinigungsmitteln auf den behandelten Flächen zusätzlich einen schädigenden Einfluss ausüben.

Aus der Patentschrift DE 198 11587 ist ein Verfahren zur thermischen Desinfektion von ebenen und raumbildenden Flächen bekannt, wobei Wasserdampf einem erfindungsgemäßen Desinfektionskopf zugeführt und anschließend als Kondensgut sterilisiert wird.

In diesem Desinfektionsverfahren, das ganz auf chemische Wirkstoffe verzichtet, sind Reinigungs- und Desinfektionskomponente sachlich und zeitlich getrennt. Es wäre aus ökonomischen und ökologischen Gründen jedoch wünschenswert. besonders bei größeren Oberflächen, wie z.B. Bodenflächen in Krankenhäusern, eine Reinigung und Desinfektion in möglichst einem Arbeitsgang zu erreichen.

Die Erfindung stellt sich daher die Aufgabe, die bekannten Reinigungssysteme zur mechanischen Flächenreinigung um eine ausreichend wirksame und zuverlässige Desinfektion ohne chemische Wirkstoffe zu ergänzen und damit ein Verfahren zu schaffen, in dem Reinigungsschritt und Desinfektionsschritt möglichst wirtschaftlich und zuverlässig zusammenarbeiten.

Zur Lösung dieser Aufgabe ist erfindungsgemäß vorgesehen, ein fahrbares Gerät mit einer Dampfvorrichtung auszustatten, wobei das Gerät während des Fahrbetriebes zunächst mechanisch reinigt, etwa durch eine Anordnung rotierender Bürsten und Vorrichtungen zur Aufnahme von Schmutz und Staubpartikeln im vorderen Geräte-Bereich, und dann durch die Dampfvorrichtung im rückwärtigen Bereich des Gerätes die nachfolgende thermische Desinfektion der vorgereinigten Oberflächen gemäß dem kennzeichnenden Merkmal des Hauptanspruchs erfolgt.

Bei einem erfindungsgemäßen Gerät können die Reinigungs- und Desinfektionsleistungen durch eine vom Benutzer wählbare Geschwindigkeit variiert werden. Dadurch wird erreicht, dass Reinigungs- und Desinfektionsintervalle an die örtlich gegebenen Raum-, Material- und Temperaturunterschiede angepasst werden können.

Ein wichtiger Vorzug besteht in der größeren Auftragsicherheit des Dampfes aus reinem Wasser gegenüber chemischen Wirkstoffen oder entsprechenden Gemischen. Ein weiterer Vorteil besteht in einer gründlicheren Reinigung, da durch den Wegfall sämtlicher Chemikalien eine statische Entladung der nur mit reinem Wasserdampf behandelten Oberflächen erfolgt Da mit der Kondensation des Dampfes auf der Oberfläche gleichzeitig eine Abkühlung durch entstehende Verdunstungskälte gegeben ist, ist eine thermische Überlastung bei allen dynamischen Bewegungsabläufen so gut wie ausgeschlossen.

In weiterer Ausgestaltung des Erfindungsgedankens wird die Dampfdesinfektionsvorrichtung mit mehreren Desinfektionsköpfen versehen, die zudem unterschiedliche Abstände zu der zu desinfizierenden Oberfläche aufweisen. Es zeigte sich, dass bei größeren mobilen Geräten eine möglichst gleichmäßige Verteilung von Reinigung und Desinfektion der behandelten Flächen dann erreicht wird, wenn Bürstfläche der rotierenden Bürsten und die Mündungsaußenfläche der Dampfdesinfektionsvorrichtung etwa gleich groß sind.

Sinnvoll ist somit, die Anzahl der Reinigungsbürsten und deren mechanische Wirkfläche an die Zahl und die Fläche der Düsenstöcke tragenden Desinfektionsköpfe anzupassen. Der Vorteil unterschiedlicher Abstände von Mündungsfläche zu der zu desinfizierenden Oberfläche besteht in der Möglichkeit, strukturierte Oberflächen, wie z.B. textile Bodenbeläge, gleichzeitig reinigen und desinfizieren zu können und in der Möglichkeit, mit verschiedenen Temperaturen das jeweils erforderliche Desinfektionsergebnis erzielen zu können. So kann beispielsweise ein großflächiger Desinfektionskopf mit einem größeren Abstand zur Desinfektionsoberfläche dort eingesetzt werden, wo relativ hitzeempfindliche Mikroorganismen großflächig angesiedelt sind, etwa im Küchenbereich.

Von Vorteil ist ferner die Beweglichkeit der Düsenstöcke, um durch verschiedene Neigungswinkel der Mündungsflächen die Durchdringungsfähigkeit des Dampfes auf textilen Oberflächen, wie z.B. Matratzen zu erhöhen.

Das Gerät trägt umlaufend ein Kunststoff- oder Bürstenband, um ein unerwünschtes Verwirbeln der Mikroorganismen zu verhindern.

Der rückwärtige Bereich des Gerätes weist dabei eine geschlossene Bodenplatte auf. Die Bodenplatte hat Durchbrüche für einen oder mehrere Düsenstöcke und eine Absaugvorrichtung.

Somit bilden Bodenplatte, das umlaufende Kunststoff- oder Bürstenband mit der zu desinfizierenden Fläche eine flache Dampfkammer. Hieraus wird durch eine geeignete Absaugvorrichtung das entstandene Dampfkondensat abgesaugt.

Weitere Vorteile und Einzelheiten der Erfindung ergeben sich aus der nachfolgenden Beschreibung einiger Ausführungsbeispiele, sowie anhand der schematisierten Zeichnungen.

Es zeigen:
- Fig.1: einen Querschnitt eines kombinierten Reinigungs- und Desinfektionsapparates mit einer Anordnung rotierender Bürsten, eine Absaugvorrichtung zur Schmutzaufnahme, eine Dampfdesinfektionsund Sterilisationsvorrichtung, wobei deren Dampfauslässe in mehrere Düsenstöcke führen, die unterschiedliche Abstände zur Bodenfläche halten, sowie eine Absaugvorrichtung zur Aufnahme des Kondensats.
- Fig. 2: die Ansicht eines fahrbaren Gerätes von unten mit einer Anordnung verschieden ausgestalteter Düsenstöcke und der Ausprägung des hinteren Geräteteils als beweglicher Desinfektionskopf mit Dampfkammer und Absaugglocke.
- Fig. 3: den Querschnitt verschieden ausgestalteter Desinfektionsköpfe, wobei die Mündungsaußenflächen der darin befindlichen Düsenstöcke einen unterschiedlichen Abstand zur Bodenfläche aufweisen.

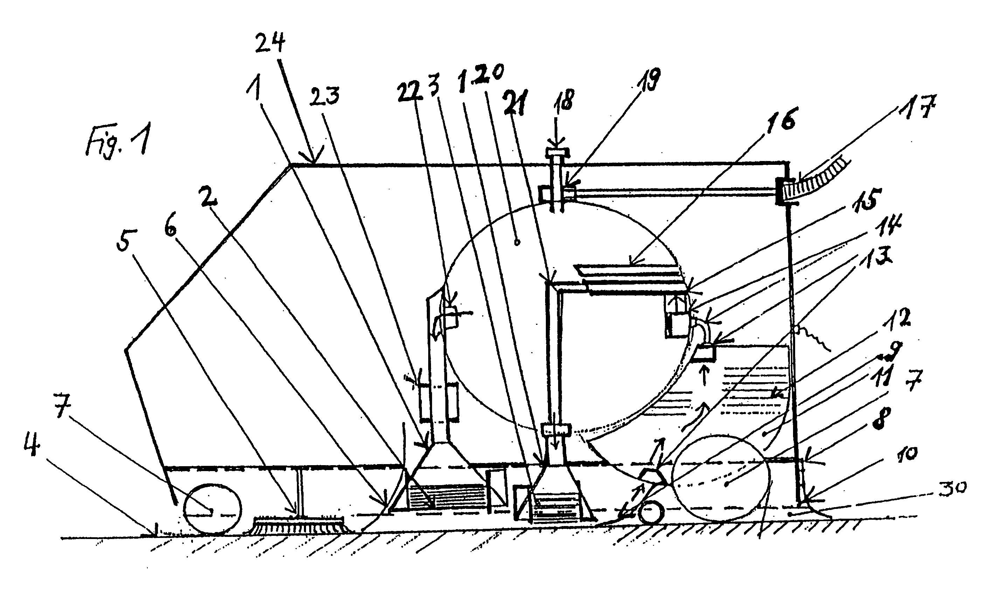

In Fig. 1 ist ein fahrbares Gerät dargestellt, das im vorderen Bereich rotierende Bürsten 5 und weitere Reinigungsvorrichtungen 6 aufweist und im hinteren Bereich eine Dampfdesinfektionsvorrichtung mit Ausgangsverdampferzelle 20,

Dampfauslassventilen 22, außerhalb 23 und innerhalb 21 der Verdampferzelle 20 verlaufenden Zuführungen 23, 21 zu verschieden ausgestalteten Desinfektionsköpfen 1 aufweist.

Das fahrbare Gerät 24 läuft auf Rädern oder Rollen 7, die einen Abstand 30 zwischen der zu reinigenden und zu desinfizierenden Fläche 4 und der unteren Bodenplatte 10 herstellen, wobei der Abstand vom Benutzer unterschiedlich eingestellt werden kann.

Der untere Bereich des Gerätes ist von einem Kunststoff- oder Bürstenband 8 umgeben, das zusammen mit der zu desinfizierenden Fläche 4 und der unteren Bodenplatte 10 mit darin integrierten Düsenstöcken 3 eine im wesentlichen geschlossene Dampfkammer 30 bildet.

Der aus einer oder mehreren Düsenstöcken 3 emittierte, reine Wasserdampf kondensiert auf der zu desinfizierenden Fläche 4 und wird über eine Absaugpumpe 13 zunächst in einen Auffangbehälter 11, der im Inneren mit goldbeschichteten KühlLamellen 12 ausgestattet ist, abgesaugt und von dort aus über eine weitere Absaugpumpe 13 über eine Zuführung 14 in die Ausgangsverdampferzelle 20 und/oder ein beheiztes Rohr 15 eingebracht.

Das beheizte Rohr 15 verläuft im Inneren der Ausgangsverdampferzelle 20 parallel zu einem weiteren Heizelement 16 und mündet in eine sich verjüngende Zuführung 21, wobei die Teilmenge des rückgeführten Kondensats aus dem Auffangbehälter 11 bereits in dem beheizten Rohr 15 verdampft, mit zunehmender Fließgeschwindigkeit durch die sich verjüngende Zuführung 21 geführt wird und als möglichst wassertröpfchenfreier Dampf den Düsenstock 3 bei laminarer Strömung verlässt.

Ein weiterer Desinfektionskopf 1 wird über ein Dampfauslassventil 22 über eine Zuführung mit einer regelbaren Heizung 23 direkt aus der Ausgangsverdampferzelle 20 gespeist. Die Zufuhr von Wasser erfolgt über einen permanenten Wasseranschluss 17 oder einen Wassereinlassstutzen 18, wobei beide Kaltwasserzuführungen durch ein angelegtes elektromagnetisches Feld verlaufen, vorzugsweise durch einen nach dem Stand der Technik bekannten Haftmagneten 19 zur Wasserentkalkung.

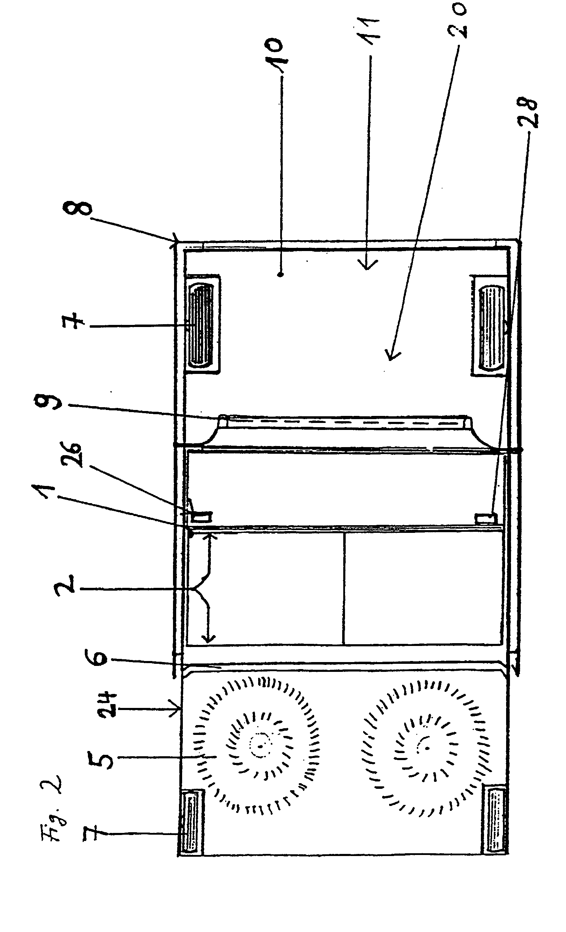

Fig. 2 zeigt das fahrbare Gerät 24 von unten, mit zwei rotierenden Bürsten 5, einer über die ganze Breite des Gerätes verlaufenden Absaugvorrichtung 6 für Schmutz, die Mündungsfläche 2 von insgesamt drei Düsenstöcken 3, wobei die beiden vorderen, geteilten Düsenstöcke 3 aus der Ausgangsverdampferzelle 20 gespeist werden, der hintere, ungeteilte Düsenstock 3 aus einem hier nicht näher dargestellten beheiztem Rohr 15, das durch die Ausgangsverdampferzelle verläuft.

Die Öffnungsschlitze der Absaugvorrichtung 9 verlaufen in einem geeigneten Abstand parallel zu den Düsenstöcken 3 und führen das abgesaugte Kondensgut in den Auffangbehälter 11. Die untere Bodenplatte 10 wird von einem Bürstenkranz 8 umfaßt und kann vom Benutzer in einem veränderbaren Abstand zur Bodenfläche 4 eingestellt werden, wobei ein Ultraschallentfernungsmesser 26 und ein Thermofühler 28 am Desinfektionskopf 1 angebracht sind.

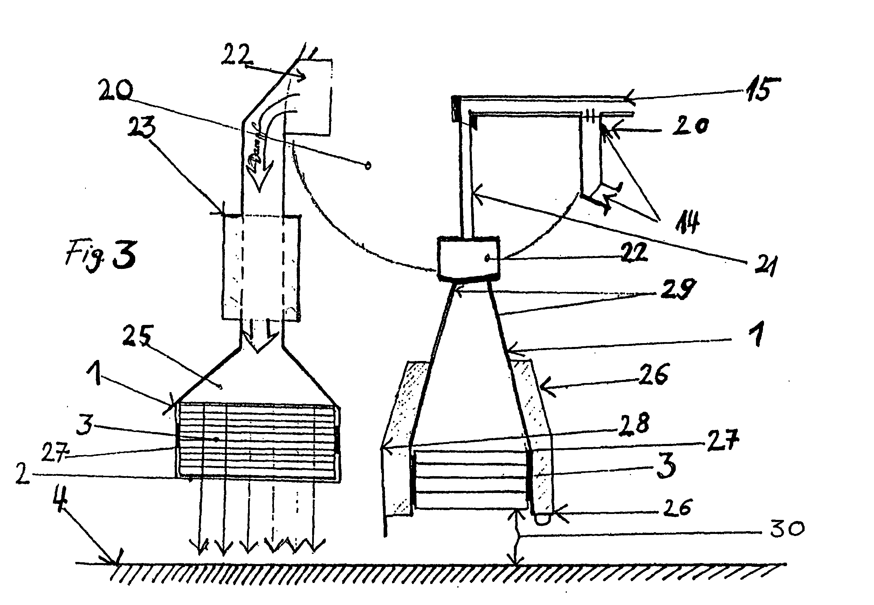

Fig. 3 zeigt zwei verschieden ausgestaltete Desinfektionsköpfe 1, wobei der vordere Desinfektionskopf 1 aus der Ausgangsverdampferzelle 20 über ein Dampfauslassventil 22 mit Wasserdampf gespeist wird, der über eine vom Benutzer regelbare Heizung 23 zusätzlich erhitzt wird.

Der überhitzte Wasserdampf wird zur Reduzierung von Wassertröpfchen über ein Goldfasergespinst 25 im vorderen Teil des Desinfektionskopfes 1 geführt und verlässt den edelmetallbeschichteten Düsenstock 3 bei laminarer Strömung. Der Desinfektionskopf 1 und der Düsenstock 3 sind hierbei durch einen Steckanschluss 27 verbunden, wodurch ein Auswechseln des Düsenstocks 3 erleichtert wird. Die Stärke des Düsenstocks spiegelt in diesem Ausführungsbeispiel den Abstand zwischen Mündungsfläche 2 und der zu desinfizierenden Fläche 4. Der hintere Desinfektionskopf 1 wird gespeist mit Wasserdampf, der in einem beheizten Rohr 15 aus dem rückgeführten Kondensat gebildet wird. Die Fließgeschwindigkeit des Wasserdampfes wird zusätzlich durch den Durchmesser einer sich verjüngenden Zuführung 21 geregelt.

Zweckmäßig ist eine Edelmetall-Beschichtung 29, vorzugsweise mit Gold, im Inneren des Desinfektionskopfes 1.

Die Abstände der Teilscheiben des eingesetzten Düsenstocks 3 sind veränderbar und können Fließgeschwindigkeit und Temperatur des Wasserdampfes im hinteren Bereich des Desinfektionskopfes 1 in gewünschter Weise verändern.

In weiterer Ausgestaltung der Erfindung sind ein Ultraschallentfernungssensor 26, sowie ein Thermofühler 28 am Desinfektionskopf 1 angebracht, die über eine Niveauregulierung der Räder 7 einen beliebigen, vom Benutzer festzulegenden Abstand zwischen Bodenplatte 10 und der zu desinfizierenden Bodenfläche 4 steuern können.

Eine besonders vorteilhafte Ausgestaltung ist eine feste Installation der erfindungsgemäßen Vorrichtung, wobei anstelle der zu desinfizierenden Bodenfläche 4 ein Fließband mit darauf befindlichem Desinfektionsgut, z.B. zu desinfizierenden Lebensmitteln, in einem geeigneten Abstand an der Mündungsfläche der Düsenstöcke 3 vorbeigeführt wird.

Die effektive Dampfeinwirkzeit eines Düsenstockes fällt im wesentlichen mit dem Kondensationsschritt auf dem Desinfektionsobjekt zusammen.

## Patentansprüche

1. Gerät zur Reinigung und Desinfektion von Oberflächen, umfassend eine mechanische Reinigungsvorrichtung (5, 6) und eine wasserdampfaufbringende Desinfektionsvorrichtung (1, 3), **dadurch gekennzeichnet, dass** in dem Gerät zur Reinigung und nachfolgenden Desinfektion in Bezug auf seine normale Bewegungsrichtung der Reinigungsvorrichtung (5, 6) die Desinfektionsvorrichtung (1, 3) nachgeordnet ist, die eine Ausgangsverdampferzelle (20) und einen daran angeschlossenen Düsenstock (3) mit einem Desinfektionskopf (1) zur Aufbringung des Wasserdampfes sowie einen Auffangbehälter (11) zur Aufnahme des von einer Absaugvorrichtung (9, 13) aufgenommenen Dampfkondensats und dessen Rückführung zu der Ausgangsverdampferzelle (20) enthält.

2. Gerät nach Anspruch 1, **dadurch gekennzeichnet, dass** der rückwärtige Bereich des Gerätes (24) eine geschlossene Bodenplatte (10) aufweist und dass die Bodenplatte Durchbrüche für einen oder mehrere Düsenstöcke (3) und eine Absaugvorrichtung (9) aufweist, und dass die Bodenplatte (10) zu der zu desinfizierenden Fläche (4) umlaufend ein Kunststoffband oder einen Bürstenkranz (8) besitzt und so mit der zu desinfizierenden Fläche (4) eine geschlossene Dampfkammer (30) bildet.

3. Gerät nach Anspruch 1 oder 2, **dadurch gekennzeichnet, dass** die Abstände zwischen der Mündungsfläche (2) der Düsenstöcke (3) und der zu desinfizierenden Fläche (4) zwischen 5 und 500 mm liegen und veränderbar sind.

4. Gerät nach einem der Ansprüche 1 bis 3, **dadurch gekennzeichnet, dass** die Abstände der Teilscheiben der Düsenstöcke (3) zwischen 1 und 50 mm liegen und veränderbar sind.

5. Gerät nach einem der Ansprüche 1 bis 4, **dadurch gekennzeichnet, dass** die Mündungsflächen (2) der Düsenstöcke (3) verschiedene Neigungswinkel zu der zu desinfizierenden Fläche (4) aufweisen, wobei ein oder mehrere Düsenstöcke (3) auch kaskadenförmig angeordnet sein können.

6. Gerät nach einem der Ansprüche 1 bis 5, **dadurch gekennzeichnet, dass** die Dampfzuführung (21) zu den Desinfektionsköpfen (1) im Inneren eine Metall-Beschichtung (29), vorzugsweise aus Gold, aufweist und zusätzlich durch eine regelbare Heizung (23) beheizbar ist.

7. Gerät nach einem der Ansprüche 1 bis 6, **dadurch gekennzeichnet, dass** die Kaltwasserzufuhr (17, 18) zur Ausgangsverdampferzelle (20) durch ein elektromagnetisches Feld geführt ist.

8. Gerät nach einem der Ansprüche 1 bis 7, **dadurch gekennzeichnet, dass** zur Bildung eines geschlossenen Wasserdampf-Kreislaufs der Auffangbehälter (11) über ein beheiztes Rohr (15) mit der Ausgangsverdampferzelle (20) verbunden ist.

9. Gerät nach Anspruch 8, **dadurch gekennzeichnet, dass** ein Desinfektionskopf (1) direkt aus dem im beheizten Rohr (15) zu Wasserdampf erhitzten Kondensat gespeist wird.

10. Gerät nach Anspruch 8 und 9, **dadurch gekennzeichnet, dass** eine Teilmenge aus dem Kondensat des Auffangbehälters (11) in das beheizte Rohr (15) geführt wird, und eine weitere Teilmenge über eine Zuführung (14) in die Ausgangsverdampferzelle (20) zurückgeführt wird.

11. Gerät nach Anspruch 8, **dadurch gekennzeichnet, dass** das beheizte Rohr (15) sowohl das rückgeführte Kondensat aus dem Auffangbehälter (11), als auch den Wasserdampf der Ausgangsverdampfenzelle (20) erhitzt.

## Claims

1. Apparatus for cleaning and disinfecting surfaces, including a mechanical cleaning device (5, 6) and a steam disinfecting device (1, 3), **characterised in that** the disinfecting device (1, 3) is arranged downstream of the cleaning device (5, 6) in the apparatus for cleaning and subsequent disinfecting with respect to its normal direction of movement and comprises an initial evaporator cell (20) and a subsequent nozzle assembly (3) with a disinfecting head (1) for applying the steam, as well as a collecting tank (11) for receiving the steam condensate taken up by a suction device (9, 13) and returning it to the initial evaporator cell (20).

2. Apparatus according to claim 1, **characterised in that** the rear region of the apparatus (24) has a closed base plate (10), that the base plate has openings for one or more nozzle assemblies (3) and a suction device (9) and that the base plate (10) has a plastic band or a ring of brushes (8) running around the surface (4) to be disinfected, thereby forming a closed steam chamber (30) together with the surface (4) to be disinfected.

3. Apparatus according to claim 1 or claim 2, **characterised in that** the distances between the surfaces (2) of the orifices of the nozzle assemblies (3) and the surface (4) to be disinfected are between 5 and 500 mm and are variable.

4. Apparatus according to one of claims 1 to 3, **characterised in that** the distances between the dividing plates of the nozzle assemblies (3) are between 1 and 50 mm and are variable.

5. Apparatus according to one of claims 1 to 4, **characterised in that** the surfaces (2) of the orifices of the nozzle assemblies (3) have differing angles of inclination relative to the surface (4) to be disinfected, wherein one or more nozzle assemblies (3) may also have a cascade arrangement.

6. Apparatus according to one of claims 1 to 5, **characterised in that** the line (21) for feeding steam to the disinfecting heads (1) is provided in its interior with a metal coating (29), preferably of gold, and can additionally be heated by means of a controllable heater (23).

7. Apparatus according to one of claims 1 to 6, **characterised in that** the line (17, 18) for feeding cold water to the initial evaporator cell (20) is guided through an electromagnetic field.

8. Apparatus according to one of claims 1 to 7, **characterised in that** the collecting tank (11) is connected to the initial evaporator cell (20) by means of a heated tube (15) in order to form a closed steam circuit.

9. Apparatus according to claim 8, **characterised in that** a disinfecting head (1) is supplied directly from the condensate heated to form steam in the heated tube (15).

10. Apparatus according to claims 8 and 9, **characterised in that** part of the condensate from the collecting tank (11) is guided into the heated tube (15) and another part is returned to the initial evaporator cell (20) via a feed line (14).

11. Apparatus according to claim 8, **characterised in that** the heated tube (15) heats both the recycled condensate from the collecting tank (11) and the steam from the initial evaporator cell (20).

## Revendications

1. Appareil destiné au nettoyage et à la désinfection de surfaces, comprenant un dispositif de nettoyage (5, 6) mécanique et un dispositif de désinfection (1, 3) par application de vapeur d'eau, **caractérisé en ce que** le dispositif de désinfection (1, 3) est disposé à la suite du dispositif de nettoyage (5, 6) dans le sens normal de déplacement de l'appareil, pour procéder au nettoyage et ensuite à la désinfection, lequel dispositif de désinfection contient une cellule de vaporisation de départ (20) et un porte-buse (3), qui est relié à celle-ci et qui comporte une tête de désinfection (1) pour l'application de la vapeur d'eau, ainsi qu'un récipient collecteur (11) prévu pour recevoir le condensat de vapeur, recueilli par un dispositif d'aspiration (9, 13), et pour le réinjecter dans la cellule de vaporisation de départ (20).

2. Appareil selon la revendication 1, **caractérisé en ce que** la zone arrière de l'appareil (24) présente une plaque de fond (10) fermée, et **en ce que** la plaque de fond présente des ouvertures traversantes pour un ou plusieurs porte-buse(s) (3) et pour un dispositif d'aspiration (9), et **en ce que** la plaque de fond (10) possède une bande de matière plastique ou une couronne à brosses (8), située tout autour par rapport à la surface à désinfecter (4), de façon à former ainsi, avec la surface à désinfecter (4), une chambre fermée à vapeur (30).

3. Appareil selon la revendication 1 ou 2, **caractérisé en ce que** les distances entre la face d'embouchure (2) des porte-buses (3) et la surface à désinfecter (4) sont comprises dans la fourchette de 5 à 500 mm et sont modifiables.

4. Appareil selon l'une des revendications 1 à 3, **caractérisé en ce que** les espacements des disques diviseurs des porte-buses (3) sont compris dans la fourchette de 1 à 50 mm et sont modifiables.

5. Appareil selon l'une des revendications 1 à 4, **caractérisé en ce que** les faces d'embouchure (2) des porte-buses (3) présentent des angles différents d'inclinaison par rapport à la surface à désinfecter (4) si bien qu'un ou plusieurs porte-buses (3) peuvent également être disposés en cascade.

6. Appareil selon l'une des revendications 1 à 5, **caractérisé en ce que** le conduit d'amenée de vapeur (21) aux têtes de désinfection (1) présente, à l'intérieur, un revêtement métallique (29), de préférence en or, et **en ce qu'**il peut en outre être chauffé par un élément de chauffage (23) réglable.

7. Appareil selon l'une des revendications 1 à 6, **caractérisé en ce que** l'alimentation en eau froide (17, 18) de la cellule de vaporisation de départ (20) est guidée par un champ électromagnétique.

8. Appareil selon l'une des revendications 1 à 7, **caractérisé en ce que**, pour former un circuit fermé de vapeur d'eau, le récipient collecteur (11) est relié à la cellule de vaporisation de départ (20) par l'intermédiaire d'un tuyau chauffé (15).

9. Appareil selon la revendication 8, **caractérisé en ce qu'**une tête de désinfection (1) est alimentée directement à partir du condensat chauffé pour transformation en vapeur d'eau, qui est contenu dans le tuyau chauffé (15).

10. Appareil selon les revendications 8 et 9, **caractérisé en ce qu'**une partie du condensat présent dans le récipient collecteur (11) est acheminée dans le tuyau chauffé (15), et **en ce qu'**une autre partie est réinjectée, par l'intermédiaire d'un conduit d'amenée (14), dans la cellule de vaporisation de départ (20).

11. Appareil selon la revendication 8, **caractérisé en ce que** le tuyau chauffé (15) chauffe aussi bien le condensat, à réinjecter à partir du récipient collecteur (11), que la vapeur d'eau de la cellule de vaporisation de départ (20).
